# EUROPEAN PATENT APPLICATION

(11) **EP 4 607 197 A1**
(43) Date of publication of application: **27.08.2025**
(21) Application number: 23879838.3
(22) Date of filing: 18.10.2023
(51) Int. Cl.: G01N 33/564, C07K 16/00, C07K 16/28, C12Q 1/02

(54) **EXAMINATION METHOD FOR IMMUNE-RELATED ADVERSE EVENT ENTERITIS**

(30) Priority: 19.10.2022 JP 2022167752
(71) Applicant: Kyoto University, Kyoto-shi, Kyoto 606-8501 (JP); Kinki University, Higashiosaka-shi, Osaka 577-8502 (JP)
(72) Inventor: SHIOKAWA, Masahiro, Kyoto-shi, Kyoto 606-8501 (JP); YOKODE, Masataka, Kyoto-shi, Kyoto 606-8501 (JP); KUWADA, Takeshi, Kyoto-shi, Kyoto 606-8501 (JP); MURAMOTO, Yuya, Kyoto-shi, Kyoto 606-8501 (JP); KAWAKAMI, Hisato, Osakasayama-shi, Osaka 589-8511 (JP); NAKAGAWA, Kazuhiko, Osakasayama-shi, Osaka 589-8511 (JP)
(74) Representative: Hoffmann Eitle
(86) International application number: PCT/JP2023/037734
(87) International publication number: WO 2024/085195

(57) **Abstract**

Provided is an examination method for irAE enteritis, said examination method comprising a detection step for detecting, as an indicator of ulcerous colitis-like irAE enteritis, an antibody that immunologically reacts with a fragment of, or the entirety of, integrin αvβ6 in a specimen.

## Description

### TECHNICAL FIELD

The present invention relates to a method of testing for immune-related adverse event (irAE) enteritis, a kit for testing for irAE enteritis, and the like.

### BACKGROUND ART

When cancer patients receive treatment with immune checkpoint inhibitors (ICIs), their immune system can go out of control as a result of releasing brakes on the immune system, resulting in irAEs. There are a variety of irAEs that can occur in various parts of the body. One of irAEs is enteritis (hereinafter referred to as irAE enteritis). The irAE enteritis is one of serious side effects of ICI therapy that can lead to patient death if treatment is delayed.

Since the irAE enteritis is essentially caused by an excessive immune response that occurs when immunosuppression is released by ICI, improvement can be seen by temporarily discontinuing administration of ICI and administering treatment with immunosuppressants such as steroids. On the other hand, immunosuppression by the use of immunosuppressants is generally contraindicated for patients having infectious enteritis caused by bacteria, etc. Furthermore, for enteritis developed as a side effect of combined therapy of an ICI and a chemotherapy agent, treatments should be varied depending on whether the enteritis is irAE enteritis caused by the ICI or enteritis caused by the chemotherapy agent. If an immunosuppressant is administered for enteritis caused by a chemotherapy agent, the symptoms of the enteritis are not improved, and further the symptoms may be worsened depending on the cause of the enteritis. Therefore, it is necessary to determine whether or not the enteritis is irAE that requires the use of immunosuppressants in order to administer appropriate treatment. However, since irAE enteritis is essentially diagnosed by exclusion, it is currently difficult to make a definitive diagnosis of irAE enteritis. In addition, since the therapeutic effect of ICI in cancer patients is brought about by the activation of immune responses of the patients, administration of immunosuppressants opposing the immune response activation may weaken the therapeutic effect of ICI. This is one reason why people are hesitant to accept treatment with immunosuppressants. On the other hand, delay in administration of immunosuppressants for irAE enteritis results in severe irAE enteritis. Therefore, in clinical practice, there is an urgent need to develop a method of determining whether or not such enteritis is caused by irAE.

Furthermore, not all patients who develop irAE enteritis require treatment with immunosuppressants such as steroids or anti-TNFα antibodies. For example, patients having relatively mild irAE enteritis without mucosal sloughing do not require administration of immunosuppressants, and discontinuation of administration of ICI for a while may be sufficient. Therefore, there is also a need for a method of determining whether irAE enteritis requires immunosuppressants.

Diagnostic methods comprising using biomarkers as indicators have been devised as techniques for diagnosing whether or not a patient is suffering from a particular type of enteritis. For example, it has been reported that integrin αvβ6 autoantibodies in a specimen are detected as an indicator of ulcerative colitis or primary sclerosing cholangitis (Patent Document 1). However, to date, autoantibodies that can serve as an indicator of irAE enteritis have never been reported.

### PRIOR ART DOCUMENT

### PATENT DOCUMENT

Patent Document 1: WO2020/1416008

### SUMMARY OF THE INVENTION

### PROBLEMS TO BE SOLVED BY THE INVENTION

An object of the present invention is development of a method of testing for irAE enteritis, in particular a method of determining whether or not enteritis developed in a cancer patient undergoing ICI treatment is irAE enteritis, or a method of determining whether or not a cancer patient who is about to undergo ICI treatment is likely to develop irAE enteritis in the future.

### SOLUTIONS TO THE PROBLEMS

In order to attain the above-described object, the present inventors intensively studied. As a result, the present inventors surprisingly found that, among cancer patients who received administration of an ICI and developed enteritis, patients having ulcerative colitis-like clinical conditions had significantly high blood levels of autoantibodies against integrin αvβ6, and that the symptoms of enteritis were improved when an immunosuppressant was administered to such patients. Furthermore, the present inventors surprisingly found that patients who developed ulcerative colitis-like irAE enteritis had significantly high blood levels of autoantibodies against integrin αvβ6 even before receiving ICI treatment. Thus, the present invention was completed.

Therefore, the present invention provides the following aspects.
(1) A method of testing for irAE enteritis, the method comprising:
   detecting an antibody that immunologically reacts with a fragment or an entirety of integrin αvβ6 in a sample as an indicator of ulcerative colitis-like irAE enteritis.
(2) The method according to (1), wherein the fragment or entirety of integrin αvβ6 is used as an antigen and an antibody that immunologically reacts with the antigen is detected.
(3) The method according to (1) or (2), wherein the sample is a blood sample.
(4) The method according to any one of (1) to (3), wherein the indicator is an indicator of severe ulcerative colitis-like irAE enteritis.
(5) A kit for testing for irAE enteritis, comprising a fragment or an entirety of integrin αvβ6.
(6) The kit according to (5), further comprising an antibody for detection.
(7) The kit according to (5) or (6), further comprising a positive standard solution and/or a negative standard solution.
(8) The kit according to any one of (5) to (7), wherein the fragment or entirety of integrin αvβ6 is immobilized on a solid phase.
(9) The method according to any one of (1) to (4), wherein the method is for determining whether or not enteritis developed in a patient receiving immune checkpoint inhibitor (ICI) treatment is ulcerative colitis-like irAE enteritis,
   wherein the sample is derived from a patient receiving ICI treatment, and when an antibody titer of the antibody that immunologically reacts with a fragment or an entirety of integrin αvβ6 detected in the sample is higher than an antibody titer of the antibody detected in a control sample, the enteritis developed in the patient is determined to be ulcerative colitis-like irAE enteritis,
   wherein the patient is a human or a non-human animal, preferably a human.
(10) The method according to (9), wherein when an antibody titer of the antibody that immunologically reacts with a fragment or an entirety of integrin αvβ6 detected in the sample is higher than an antibody titer of the antibody detected in a control sample, the enteritis developed in the patient is determined to be severe ulcerative colitis-like irAE enteritis.
(11) The method according to (9) or (10), wherein the patient is a patient receiving combined treatment with an ICI and a cancer chemotherapeutic drug other than an ICI.
(12) The method according to any one of (1) to (4), wherein the method is for predicting a risk of developing ulcerative colitis-like irAE enteritis in a patient,
   wherein the sample is derived from a patient, and when an antibody titer of the antibody that immunologically reacts with a fragment or an entirety of integrin αvβ6 detected in the sample is higher than an antibody titer of the antibody detected in a control sample, the patient is determined to be at risk of developing ulcerative colitis-like irAE enteritis,
   wherein the patient is a human or a non-human animal, preferably a human.
(13) The method according to (12), wherein when an antibody titer of the antibody that immunologically reacts with a fragment or an entirety of integrin αvβ6 detected in the sample is higher than an antibody titer of the antibody detected in a control sample, the patient is determined to be at risk of developing severe ulcerative colitis-like irAE enteritis.
(14) The method according to (12) or (13), wherein the patient is a patient prior to ICI treatment or a patient receiving ICI treatment.
(15) A method of testing for irAE enteritis, the method comprising:
   detecting an antibody that immunologically reacts with a fragment or an entirety of integrin αvβ6 in a sample as an indicator of enteritis for which discontinuation of an ICI is recommended.
(16) The method according to (15), wherein the indicator is an indicator of enteritis for which discontinuation of an ICI and administration of one or more immunosuppressants are recommended.
(17) The method according to (15) or (16), wherein the fragment or entirety of integrin αvβ6 is used as an antigen and an antibody that immunologically reacts with the antigen is detected.
(18) The method according to any one of (15) to (17), wherein the sample is a blood sample.
(19) The method according to any one of (15) to (18), wherein the method is for determining whether or not enteritis developed in a patient receiving ICI treatment is enteritis for which discontinuation of an ICI is recommended,
   wherein the sample is derived from a patient receiving ICI treatment, and when an antibody titer of the antibody that immunologically reacts with a fragment or an entirety of integrin αvβ6 detected in the sample is higher than an antibody titer of the antibody detected in a control sample, the enteritis developed in the patient is determined to be enteritis for which discontinuation of an ICI is recommended.
(20) The method according to (19), wherein when an antibody titer of the antibody that immunologically reacts with a fragment or an entirety of integrin αvβ6 detected in the sample is higher than an antibody titer of the antibody detected in a control sample, the enteritis developed in the patient is determined to be enteritis for which discontinuation of an ICI and administration of one or more immunosuppressants are recommended.
(21) The method according to (19) or (20), wherein the patient is a patient receiving combined treatment with an ICI and a cancer chemotherapeutic drug other than an ICI.
(22) The method according to any one of (15) to (18), wherein the method is for predicting a risk of developing enteritis for which discontinuation of an ICI is recommended in a patient,
   wherein the sample is derived from a patient, and when an antibody titer of the antibody that immunologically reacts with a fragment or an entirety of integrin αvβ6 detected in the sample is higher than an antibody titer of the antibody detected in a control sample, the patient is determined to be at risk of developing enteritis for which discontinuation of an ICI is recommended.
(23) The method according to (22), wherein when an antibody titer of the antibody that immunologically reacts with a fragment or an entirety of integrin αvβ6 detected in the sample is higher than an antibody titer of the antibody detected in a control sample, the patient is determined to be at risk of developing enteritis for which discontinuation of an ICI and administration of one or more immunosuppressants are recommended.
(24) The method according to (22) or (23), wherein the patient is a patient prior to ICI treatment or a patient receiving ICI treatment.
(25) The kit according to any one of (5) to (8), wherein the kit is for determining whether or not enteritis developed in a patient receiving ICI treatment is ulcerative colitis-like irAE enteritis,
   wherein the patient is a human or a non-human animal, preferably a human.
(26) The kit according to (25), wherein the patient is a patient receiving combined treatment with an ICI and a cancer chemotherapeutic drug other than an ICI.
(27) The kit according to any one of (5) to (8), wherein the kit is for predicting a risk of developing ulcerative colitis-like irAE enteritis in a patient,
   wherein the patient is a human or a non-human animal, preferably a human.
(28) The method according to (27), wherein the patient is a patient prior to ICI treatment or a patient receiving ICI treatment.
(29) A method of treating ulcerative colitis-like irAE enteritis, the method comprising:
   detecting an antibody that immunologically reacts with a fragment or an entirety of integrin αvβ6 in a sample from a patient, and
   discontinuing ICI treatment and/or administering an immunosuppressant to the patient when an antibody titer of the antibody detected in a sample from the patient is higher than an antibody titer of the antibody detected in a control sample,
   wherein the patient is a human or a non-human animal receiving ICI treatment, preferably a human receiving ICI treatment.
(30) The method according to (29), which comprises discontinuing ICI treatment and administering an immunosuppressant to the patient when the antibody titer of the antibody detected in a sample from the patient is higher than an antibody titer of the antibody detected in a control sample.
(31) A method of treating enteritis developed in a patient receiving combined treatment with an ICI and a cancer chemotherapeutic drug other than an ICI, the method comprising:
   detecting an antibody that immunologically reacts with a fragment or an entirety of integrin αvβ6 in a sample from the patient,
   discontinuing the ICI in the patient and/or administering an immunosuppressant to the patient when an antibody titer of the antibody detected in the sample from the patient is higher than an antibody titer of the antibody detected in a control sample, or
   discontinuing the cancer chemotherapeutic drug other than an ICI in the patient when an antibody titer of the antibody detected in the sample from the patient is equivalent to or lower than an antibody titer of the antibody detected in a control sample or when the antibody is not detected in the sample from the patient,
   wherein the patient is a human or a non-human animal, preferably a human.
(32) The method according to (31), which comprise discontinuing the ICI in the patient and administering an immunosuppressant to the patient when the antibody titer of the antibody detected in the sample from the patient is higher than the antibody titer of the antibody detected in a control sample.
(33) The method according to any one of (29) to (32), wherein the fragment or entirety of integrin αvβ6 is used as an antigen and an antibody that immunologically reacts with the antigen is detected.
(34) The method according to any one of (29) to (33), wherein the sample is a blood sample.
(35) A method of evaluating efficacy in treatment of ulcerative colitis-like irAE enteritis, the method comprising:
   detecting an antibody that immunologically reacts with a fragment or an entirety of integrin αvβ6 in a sample obtained from a patient receiving the treatment of ulcerative colitis-like irAE enteritis.
(36) A method of diagnosing ulcerative colitis-like irAE enteritis, the method comprising:
   detecting an antibody that immunologically reacts with a fragment or an entirety of integrin αvβ6 in a sample derived from a patient, and
   determining that the patient is suffering from ulcerative colitis-like irAE enteritis when the antibody is detected,
   wherein the patient is a human or a non-human animal receiving ICI treatment, preferably a human receiving ICI treatment.
(37) The method according to (35) or (36), wherein the fragment or entirety of integrin αvβ6 is used as an antigen and an antibody that immunologically reacts with the antigen is detected.
(38) The method according to any one of (35) to (37), wherein the sample is a blood sample.
(39) A biomarker for diagnosing ulcerative colitis-like irAE enteritis, comprising an antibody that immunologically reacts with a fragment or an entirety of integrin αvβ6.
(40) A method of screening for a candidate substance for treating ulcerative colitis-like irAE enteritis, the method comprising:
   detecting an antibody that immunologically reacts with a fragment or an entirety of integrin αvβ6 in a sample obtained from an animal treated with a test substance, and
   selecting the test substance as a candidate substance for treating ulcerative colitis-like irAE enteritis when the antibody in the sample is reduced by treating the animal with the test substance.
(41) A method of obtaining an indicator of ulcerative colitis-like irAE enteritis, the method comprising:
   detecting an antibody that immunologically reacts with a fragment or an entirety of integrin αvβ6 in a sample.
(42) The method according to (40) or (41), wherein the fragment or entirety of integrin αvβ6 is used as an antigen and an antibody that immunologically reacts with the antigen is detected.
(43) The method according to any one of (40) to (42), wherein the sample is a blood sample.
(44) Use of a fragment or an entirety of integrin αvβ6 for manufacture of a test kit or a test reagent for testing for ulcerative colitis-like irAE enteritis.
(45) The use according to (44), wherein the fragment or entirety of integrin αvβ6 is immobilized on a solid phase.
(46) A fragment or an entirety of integrin αvβ6 for use in testing for ulcerative colitis-like irAE enteritis.
(47) The fragment or entirety of integrin αvβ6 according to (46), which is immobilized on a solid phase.
(48) A method of detecting an antibody that immunologically reacts with a fragment or an entirety of integrin αvβ6 in a patient, the method comprising:
   detecting the presence of the antibody in a sample derived from the patient,
   wherein the patient is a human or a non-human animal, preferably a human, and
   wherein the patient may be an animal suffering from or suspected of suffering from ulcerative colitis-like irAE enteritis.
(49) The method according to (48), wherein the fragment or entirety of integrin αvβ6 is used as an antigen, and the presence of the antibody in the sample is detected by contacting the sample with the antigen and detecting biding of the antibody to the antigen.
(50) The method according to (49), wherein the fragment or entirety of integrin αvβ6 is used as an antigen, and the presence of the antibody in the sample is detected by contacting the sample with the antigen and further with an anti-human IgG antibody, an anti-human IgA antibody, an anti-human IgM antibody and/or an anti-human IgE antibody, and detecting biding of the antibody to the anti-human IgG antibody, the anti-human IgA antibody, the anti-human IgM antibody and/or the anti-human IgE antibody,
   wherein the antigen is preferably immobilized on a solid phase.
(51) The method according to any one of (48) to (50), wherein the sample is a blood sample isolated from the patient,
   wherein the blood sample is preferably a serum sample, a plasma sample or whole blood.
(52) A method of diagnosing ulcerative colitis-like irAE enteritis in a patient, the method comprising:
   detecting the presence of an antibody that immunologically reacts with the antigen in a sample derived from the patient by contacting the sample with a solid phase carrier on which an antigen is immobilized, wherein the antigen is a fragment or an entirety of integrin αvβ6, and detecting binding of the antibody to the antigen, and
   determining that the patient is suffering from ulcerative colitis-like irAE enteritis when the presence of the antibody in the sample is detected,
   wherein the patient is a human or a non-human animal receiving ICI treatment, preferably a human receiving ICI treatment.
(53) The method according to (52), wherein the presence of the antibody in the sample is detected by contacting the sample with the antigen which is the fragment or entirety of integrin αvβ6, and further with an anti-human IgG antibody, an anti-human IgA antibody, an anti-human IgM antibody and/or an anti-human IgE antibody, and detecting biding of the antibody to the anti-human IgG antibody, the anti-human IgA antibody, the anti-human IgM antibody and/or the anti-human IgE antibody.
(54) The method according to (52) or (53), wherein the sample is a blood sample isolated from the patient, and
   wherein the blood sample is preferably a serum sample, a plasma sample or whole blood.

### EFFECTS OF THE INVENTION

The test method and test kit for irAE enteritis of the present invention makes it possible to detect the presence or absence of ulcerative colitis-like irAE enteritis or the likelihood of developing ulcerative colitis-like irAE enteritis with high sensitivity and specificity, and to detect ulcerative colitis-like irAE enteritis specifically and distinctively from enteritis caused by chemotherapy or factors other than ICI. For example, according to the present invention, it is possible to determine whether or not enteritis is ulcerative colitis-like irAE enteritis which an immunosuppressant is effective for. Therefore, according to the present invention, it is possible to determine whether enteritis developed in a patient receiving combined treatment with an ICI and a cancer chemotherapeutic drug other than an ICI was caused by the ICI or the cancer chemotherapeutic drug other than an ICI, and whether use of an immunosuppressant is effective for the enteritis or not. Thus, according to the present invention, appropriate treatment can be carried out early.

Furthermore, according to the present invention, it is possible to detect ulcerative colitis-like irAE enteritis, among irAE enteritis, specifically and distinctively from other irAE enteritis. The present invention demonstrates that anti-integrin αvβ6 antibodies are expressed at high levels in patients having ulcerative colitis-like irAE enteritis among irAE enteritis, and that administration of an immunosuppressant is effective for such patients. Therefore, according to the present invention, it is possible to provide appropriate treatment of irAE enteritis developed in cancer patients receiving ICI treatment. Particularly, according to the present invention, it is possible to diagnose ulcerative colitis-like irAE enteritis, which is one of serious irAEs that can lead to death unless aggressive therapeutic intervention is performed at an early stage, and thereby provide appropriate treatment with patients.

Furthermore, according to the present invention, it is possible to distinguish ulcerative colitis-like irAE enteritis from non-ulcerative colitis-like irAE enteritis based on antibody titer of anti-integrin αvβ6 antibody as an indicator. The ulcerative colitis-like irAE enteritis that is identified based on the antibody titer of anti-integrin αvβ6 antibodies as an indicator is cured by administration of an immunosuppressant such as a steroid or an anti-TNFα antibody, and in other words, treatment with an immunosuppressant is essential for the ulcerative colitis-like irAE enteritis. On the other hand, non-ulcerative colitis-like irAE enteritis does not necessarily require treatment with an immunosuppressant, and may be cured by other measures, for example, by simple discontinuation of an ICI and follow-up.

### BRIEF DESCRIPTION OF THE DRAWINGS

[Fig. 1] Fig. 1 shows the levels of anti-human integrin αvβ6 antibodies in the sera of patients having ulcerative colitis-like irAE enteritis (irAE colitis UC like) and patients having non-ulcerative colitis-like irAE enteritis (irAE colitis UC unlike).
[Fig. 2] Fig. 2 shows changes over time in the anti-human integrin αvβ6 antibody level (left vertical axis) and Mayo score (right vertical axis) in the serum of a patient having ulcerative colitis-like irAE enteritis who received discontinuation of an ICI and administration of a steroid.
[Fig. 3] Fig. 3 shows endoscopic images of the intestine of a patient having ulcerative colitis-like irAE enteritis before discontinuation of an ICI and administration of a steroid.
[Fig. 4] Fig. 4 shows endoscopic images of the intestine of a patient having ulcerative colitis-like irAE enteritis after discontinuation of an ICI and administration of a steroid.
[Fig. 5] Fig. 5 shows endoscopic images of the intestine of a patient having ulcerative colitis-like irAE enteritis after discontinuation of an ICI and administration of a steroid.
[Fig. 6] Fig. 6 shows changes over time in the anti-human integrin αvβ6 antibody level (left vertical axis) and Mayo score (right vertical axis) in the serum of a patient having ulcerative colitis-like irAE enteritis who received discontinuation of an ICI.
[Fig. 7] Fig. 7 shows a graph showing changes over time in the anti-human integrin αvβ6 antibody level in the serum of a patient from before the start of ICI treatment until after the onset of ulcerative colitis-like irAE enteritis, and an endoscopic image of the large intestine of the patient at the onset of ulcerative colitis-like irAE enteritis.
[Fig. 8] Fig. 8 shows the levels of anti-human integrin αvβ6 antibodies in the sera from patients with irAE enteritis and other irAEs (hepatitis, endocrine dysfunction, pneumonitis, others), cancer patients (without irAEs), and healthy volunteers.
[Fig. 9] Fig. 9 shows endoscopic images of the intestines of anti-human integrin αvβ6 autoantibody-positive and - negative irAE enteritis patients.
[Fig. 10] Fig. 10 shows how many findings of six endoscopic findings characteristic of ulcerative colitis were observed in the intestines of anti-human integrin αvβ6 autoantibody-positive and -negative irAE enteritis patients. The vertical axis indicates the number of ulcerative colitis findings observed. The horizontal axis indicates anti-human integrin αvβ6 antibody-positive and -negative samples.
[Fig. 11] Fig. 11 shows changes over time in the anti-human integrin αvβ6 antibody level (left vertical axis) and Partial Mayo score (right vertical axis) in the serum of a patient having ulcerative colitis-like irAE enteritis who received discontinuation of an ICI and administration of a steroid.

### MODE FOR CARRYING OUT THE INVENTION

### <Ulcerative colitis-like irAE enteritis>

The irAE (immune-related adverse event) enteritis is a generic term for enteritis that occurs as adverse events due to immune checkpoint inhibitors (ICIs), and has wide variety of disease states. Specifically, there are microscopic colitis-like enteritis, which has no endoscopic abnormalities and has immune cell infiltration found only when an intestinal mucosal biopsy is conducted, and so-called inflammatory bowel disease (IBD)-like enteritis, which is accompanied by severe mucosal edema, bleeding and exfoliation (erosion, ulceration). Among these, irAE enteritis resembling ulcerative colitis (UC), a type of IBD, can be fatal if aggressive therapeutic intervention is not performed. As used herein, such irAE enteritis is referred to as "ulcerative colitis-like irAE enteritis" or "UC-type irAE enteritis."

The clinical presentation of UC-type irAE enteritis is similar to the clinical presentation of UC. In typical cases, the inflammation shows diffuse involvement extending proximally from the rectum, with continuous mucosal edema, bleeding, erosions, and ulcerations, and pathologically, crypt abscesses are formed due to immune cell infiltration, and large amounts of immune cell infiltration are observed within the tissues. UC-type irAE enteritis is clearly different from UC in that UC is a disease of unknown etiology whereas UC-type irAE enteritis is caused by an ICI.

Every type of irAE enteritis including UC-type irAE enteritis is basically caused by an excessive immune response that occurs due to release from immunosuppression by an ICI, and improved by discontinuing ICI administration and administering treatment with an immunosuppressant such as a steroid. Thus, discontinuation of ICI administration is recommended for irAE enteritis. For irAE enteritis, it is preferable to discontinue the administration of an ICI and then administer an immunosuppressant. On the other hand, immunosuppression is generally contraindicated for infectious enteritis caused by bacteria, etc. Therefore, a precondition for treatment with an immunosuppressant is that enteritis occurring in a case is irAE enteritis. However, since irAE enteritis is essentially diagnosed by exclusion, it is currently difficult to make a definitive diagnosis of irAE enteritis. In addition, since the therapeutic effect of an ICI is brought about by the activation of immune responses, administration of an immunosuppressant such as a steroid may weaken the anti-tumor effect of an ICI. This is one reason why people are hesitant to accept treatment with an immunosuppressant.

The response to steroid therapy for irAE enteritis is generally good in microscopic type irAE enteritis that does not involve mucosal damages, whereas UC-type irAE enteritis may become refractory when therapeutic intervention is delayed, and administration of an anti-TNF-α antibody is sometimes required in a case where the response to a high-dose of steroid is poor. In contrast to UC, where there is no concept of cure and only "remission" is observed, UC-type irAE enteritis responsive to treatment with an immunosuppressant is often cured by a short period of the treatment, and in some cases, an ICI may be administered again. UC-type irAE enteritis involving mucosal sloughing is rarely improved only by discontinuation of an ICI, and tends to worsen over time. Therefore, it is important to diagnose irAE enteritis as quickly as possible and not miss the timing of administering an immunosuppressant.

In the past, ICIs were administered alone. However, with advances in cancer treatment, combined treatment with an ICI and a cancer chemotherapeutic drug other than an ICI has been standardly conducted. In such combined treatment, as a cause of enteritis developed during the treatment, a factor of a cancer chemotherapeutic drug other than an ICI is added. Particularly, fluoropyrimidine anticancer drugs, which is represented by 5-FU used to treat gastrointestinal cancer, are known to frequently cause diarrhea and intestinal mucosal damages. In such a case, diagnosis of irAE enteritis becomes extremely difficult. The enteritis caused by fluoropyrimidine often occurs clinically mainly in the small intestine, particularly in the ileocecal region. However, since there are exceptions, diagnosis only based on images is often difficult. Since the enteritis caused by anticancer drugs occurs due to direct damages of the intestinal mucosa, pathological conditions of concern include bacterial translocation, in which normal intestinal bacteria enter the bloodstream across the mucosal barrier, resulting in a severe infection, sepsis. Therefore, mindlessly using immunosuppressants to treat the enteritis caused by combined treatment with an ICI and a cancer chemotherapeutic drug other than an ICI may promote such infection and is not recommended. In such a case, it is necessary to observe responses to discontinuation of an anticancer drug and treatment with a broad-spectrum antimicrobial drug. On the other hand, in the case where the cause of the enteritis is an ICI, delay in administration of an immunosuppressant results in further aggravation of the enteritis. Therefore, in clinical practice, it is important to determine whether the cause of enteritis is an irAE or not.

### <2. Patients and Samples>

The "patient" targeted by the present invention is a patient who may develop irAE enteritis or who may have developed irAE enteritis, and includes cancer patients. The cancer patients are animals afflicted with cancer. The type of cancer is not particularly limited and may be any type, preferably a cancer that is an indication for ICI treatment. Examples of the cancer include, but not limited to, malignant melanoma, non-small cell carcinoma, renal cell carcinoma, classical Hodgkin's lymphoma, head and neck cancer, gastric cancer, malignant pleural mesothelioma, esophageal cancer, MSI-High colorectal cancer, urothelial cancer, MSI-High solid cancer, esophageal squamous cell cancer, Merkel cell cancer, small cell carcinoma, breast cancer, hepatocellular carcinoma, uterine cancer, cancer of unknown primary, and TMB-High cancer. The type of animal is not particularly limited and may be a human or a non-human mammal, preferably a human.

The patient may be a patient receiving ICI treatment or a patient who is going to receive ICI treatment. As used herein, the "patient receiving ICI treatment" includes a patient currently receiving ICI treatment, and a patient who is not currently receiving ICI treatment and has received ICI treatment in the past several months, e.g., about 2-6 months. The "patient who is going to receive ICI treatment" may be any patient who is going to receive ICI treatment, and includes a patient who has never received ICI treatment, and a patient who has received ICI treatment in the past, for example, a patient who received the last administration of an ICI more than one year ago.

The ICI used in the present invention is a substance that binds to an immune checkpoint molecule or a ligand molecule thereof to inhibit signal transduction in the immune mechanism and thereby remove inhibitory signals against immune cells by the immune checkpoint molecule, and preferably have the effect of restoring the ability of immune cells to attack cancer. The ICI may be selected appropriately depending on the type of cancer that the patient is suffering from. Examples of the ICI include, but not limited to, anti-PD-1 antibodies (e.g., nivolumab, pembrolizumab, etc.), anti-PD-L1 antibodies (e.g., avelumab, atezolizumab, durvalumab, etc.), and anti-CTLA4 antibodies (e.g., ipilimumab, etc.). Two or more ICIs may be administered to the patient.

The patient receiving ICI treatment may further be a patient receiving combined treatment with an ICI treatment and a cancer chemotherapeutic drug other than an ICI. As used herein, the "patient receiving combined treatment with an ICI treatment and a cancer chemotherapeutic drug other than an ICI" includes a patient currently receiving the combined treatment, and a patient who is not currently receiving the combined treatment, but has received the combined treatment in the past and received the last administration of an ICI or a cancer chemotherapeutic drug other than an ICI in the past several months, for example about 2 to 6 months.

As used herein, examples of the "cancer chemotherapeutic drug other than an ICI" include a cytotoxic anticancer drug and a molecularly targeted drug. Examples of the "cytotoxic anticancer drug" include a drug that inhibits DNA synthesis or replication in cells to suppress cell proliferation, and a drug that acts on a microtubule, a type of cytoskeleton, and inhibits cell division to suppress cell proliferation. Specific examples of the cytocidal anticancer drug include, depending on the type of cancer that the patient is suffering from, but not limited to, fluoropyrimidine anticancer drugs such as 5-FU (fluorouracil), platinum anticancer drugs such as cisplatin, oxaliplatin and carboplatin, and metabolic antagonist anticancer drugs such as gemcitabine and pemetrexed. The "molecularly targeted drug" is a drug that targets a molecule involved in the proliferation, invasion, metastasis, or the like of cancer cells to suppress the proliferation of cancer cells or inhibit the progression of cancer, and thereby has the effect of suppressing primary cancer and even cancer metastasis. Specific examples of the molecularly targeted drug include, but not limited to, antibody preparations such as Avastin, and low molecular weight compounds such as lenvatinib.

Examples of the "sample" used in the present invention include body fluids collected from the patient. Specific examples thereof include blood samples such as serum, plasma, and whole blood, and body fluid samples other than blood, such as saliva, cerebrospinal fluid, and urine. In addition to body fluids, a tissue collected from the patient, for example, a tissues collected from a site exhibiting the pathological condition of enteritis to be tested (for example, large intestine or rectum) can be used as the sample. The sample is used in the present invention in the form isolated from the patient.

### <3. Integrin αvβ6>

Integrin in the natural form is heterodimeric protein consisting of two subunit chains, an α chain and a β chain. Known α chains are α1 to α11, αv, αX, αM, αL, αD, αE, and αIIb, and known β chains are β1 to β8. There are multiple integrin isoforms with different combinations of α chains and β chains.

Integrin is present on the surface of epithelial cells and binds to extracellular matrix proteins such as laminin and fibronectin on the surface of connective tissues.

Integrin αvβ6 consists of a heterodimeric molecule containing αv as the α chain and β6 as the β chain. Integrin αvβ6 is hardly expressed in normal tissues, and is expressed on the surface of epithelial cells upon inflammatory stimulation.

In the present invention, the origin of a fragment or an entirety of integrin αvβ6 that immunologically binds to the autoantibody to be detected is not particularly limited. The fragment or entirety of integrin αvβ6 is preferably from the same species as the patient or animal to be tested, and particularly preferably a human. The nucleotide sequence information of genes encoding the αv chain and β6 chain of integrin from mammalian species including human, and the amino acid sequence information of each chain are available from known databases (GenBank, etc.). Specifically, the amino acid sequence of a preproprotein of human integrin αv chain isoform 1 is registered under GenBank Accession Number NP_002201.2 and is shown in SEQ ID NO:1. The amino acid sequence of a precursor of human integrin β6 chain is registered under GenBank Accession Number NP_000879.2 and is shown in SEQ ID NO:2. Similarly, the amino acid sequence information of integrin αv chains and β6 chains from various mammals other than a human are available from known databases (GenBank, etc.). The integrin αvβ6 may be composed of an αv chain and a β6 chain comprising amino acid sequences containing post-translational modification to one or both of the amino acid sequences of the αv chain and the β6 chain registered in a database. For example, the partial sequence from positions 1 to 30 in the amino acid sequence of SEQ ID NO:1 is a signal peptide sequence, and the amino acid sequence of the mature polypeptide of human integrin αv chain comprises the sequence from positions 31 to 1048 in the amino acid sequence of SEQ ID NO:1. Similarly, the partial sequence from positions 1 to 21 in the amino acid sequence of SEQ ID NO:2 is a signal peptide sequence, and the amino acid sequence of the mature polypeptide of the human integrin β6 chain comprises the sequence from positions 22 to 788 in the amino acid sequence of SEQ ID NO:2.

Unless otherwise specified, the integrin αvβ6 in the present invention is not limited to the native form comprising a mature or immature amino acid sequence, and may be a mutant in a form equivalent to native integrin αvβ6.

The integrin αvβ6 is not limited to a form comprising the full lengths of both native or mutant α chain and β chain comprising mature or immature amino acid sequences (i.e., the entirety of integrin αvβ6), and may be in the form of a fragment of integrin αvβ6.

The autoantibody to be detected is not limited to an antibody that immunologically reacts with a polypeptide consisting of only the amino acid sequence of a fragment or an entirety of integrin αvβ6, and may be an antibody that immunologically binds to a polypeptide comprising a fragment or an entirety of integrin αvβ6 and other peptides attached to the both chains of the integrin αvβ6 (particularly attached to their C-terminus).

The fragment of integrin αvβ6 may be an integrin dimer comprising an αv chain and a β6 chain in which at least one of the αv chain and β6 chain constituting the integrin dimer is shorter than the α or β chain of the mature or immature, native or mutant integrin αvβ6. Specific examples of the fragment of integrin αvβ6 include dimers comprising an αv chain and a β6 chain, wherein the αv chain comprises the partial sequence from Phe at position 31 to Val at position 992 of the αv chain amino acid sequence shown in SEQ ID NO:1 as the αv chain, and/or the β6 chain comprises the partial sequence from Gly at position 22 to Asn at position 707 of the β6 chain amino acid sequence shown in SEQ ID NO:2. The fragment of integrin αvβ6 preferably forms a dimer, and more preferably has binding activity to an extracellular matrix protein such as laminin or fibronectin. The binding activity to an extracellular matrix protein of the integrin αvβ6 fragment can be determined by, for example, ELISA.

The fact that the entirety or fragment of integrin αvβ6 forms a dimer can be confirmed, for example, by detection of a band corresponding to the molecular weight of the dimer when the entirety or fragment of integrin αvβ6 is subjected to SDS-PAGE in the absence of 2-mercaptoethanol and disappearance of the band corresponding to the molecular weight of the dimer when the entirety or fragment of integrin αvβ6 is subjected to SDS-PAGE in the presence of 2-mercaptoethanol.

An example of commercially available integrin αvβ6 is recombinant human integrin αvβ6 (R&D Systems, Minnesota, USA, product number 3817-AV). This recombinant human integrin αvβ6 is a dimer of an αv chain consisting of the partial sequence from Phe at position 31 to Val at position 992 of the αv chain amino acid sequence shown in SEQ ID NO:1, and a linker sequence and an acidic tail sequence added to the C-terminus, and a β6 chain consisting of the partial sequence from Gly at position 22 to Asn at position 707 of the β6 chain amino acid sequence shown in SEQ ID NO:2, and a linker sequence and a basic tail sequence added to the C-terminus.

A more specific embodiment of the αv chain constituting the entirety or fragment of integrin αvβ6 is a polypeptide selected from the group consisting of:
(I) a polypeptide comprising the amino acid sequence shown in SEQ ID NO:1 or a partial sequence of the amino acid sequence shown in SEQ ID NO:1 from Phe at position 31 to Thr at position 1048;
(II) a polypeptide functionally equivalent to the polypeptide of (I), comprising a partial sequence of the amino acid sequence shown in SEQ ID NO:1;
(III) a polypeptide functionally equivalent to the polypeptide of (I), comprising an amino acid sequence having 85% or more sequence identity with the amino acid sequence shown in SEQ ID NO:1 or a partial sequence thereof; and
(IV) a polypeptide functionally equivalent to the polypeptide of (I), comprising an amino acid sequence in which one or more amino acids are substituted, deleted, and/or added in the amino acid sequence shown in SEQ ID NO:1 or a partial sequence thereof.

The polypeptides (I) to (IV) may comprise additional amino acid sequences as well as the amino acid sequences or partial sequences defined in (I) to (IV). The additional amino acid sequence is added to at least one of the N-terminus and C-terminus, preferably to the C-terminus, of the amino acid sequences or partial sequences defined in (I) to (IV).

In (II), (III) and (IV) as described above, an example of the polypeptide functionally equivalent to the polypeptide of (I) is a polypeptide capable of forming a dimer with an integrin β6 chain (particularly preferably, a polypeptide chain consisting of the amino acid sequence shown in SEQ ID NO:2, a polypeptide chain consisting of the partial sequence from Gly at position 22 to Cys at position 788 of the amino acid sequence shown in SEQ ID NO:2, or a polypeptide chain consisting of the partial sequence from Gly at position 22 to Asn at position 707 of the amino acid sequence shown in SEQ ID NO:2), wherein the formed dimer has the ability to bind to an extracellular matrix protein such as laminin or fibronectin to which native integrin αvβ6 or commercially available integrin αvβ6 can bind.

The partial sequence in (II) as described above includes the partial sequence from Phe at position 31 to Val at position 992 of the amino acid sequence shown in SEQ ID NO:1. The partial sequences in (III) and (IV) aa described above include the partial sequence from Phe at position 31 to Thr at position 1048 of the amino acid sequence shown in SEQ ID NO:1, and the partial sequence from Phe at position 31 to Val at position 992 of the amino acid sequence shown in SEQ ID NO:1.

The sequence identity in (III) as described above is preferably 90% or more, more preferably 95% or more, even more preferably 96% or more, particularly preferably 97% or more, and most preferably 98% or more or 99% or more.

In (IV) as described above, the term "one or more" means, for example 1 to 100, preferably 1 to 50, preferably 1 to 30, preferably 1 to 20, preferably 1 to 15, preferably 1 to 10, preferably 1 to 5, preferably 1 to 4, preferably 1 to 3, preferably 1 to 2, or preferably 1.

A more specific embodiment of the β6 chain constituting the entirety or fragment of integrin αvβ6 is a polypeptide selected from the group consisting of:
(V) a polypeptide comprising the amino acid sequence shown in SEQ ID NO:2 or a partial sequence of the amino acid sequence shown in SEQ ID NO:2 from Gly at position 22 to Cys at position 788;
(VI) a polypeptide functionally equivalent to the polypeptide of (V), comprising a partial sequence of the amino acid sequence shown in SEQ ID NO:2;
(VII) a polypeptide functionally equivalent to the polypeptide of (V), comprising an amino acid sequence having 85% or more sequence identity with the amino acid sequence shown in SEQ ID NO:2 or a partial sequence thereof; and
(VIII) a polypeptide functionally equivalent to the polypeptide of (V), comprising an amino acid sequence in which one or more amino acids are substituted, deleted, and/or added in the amino acid sequence shown in SEQ ID NO:2 or a partial sequence thereof.

The polypeptides (V) to (VIII) may comprise additional amino acid sequences as well as the amino acid sequences or partial sequences defined in (V) to (VIII). The additional amino acid sequence is added to at least one of the N-terminus and C-terminus, preferably to the C-terminus, of the amino acid sequences or partial sequences defined in (V) to (VIII).

In (VI), (VII) and (VIII) as described above, an example of the polypeptide functionally equivalent to the polypeptide of (V) is a polypeptide capable of forming a dimer with an integrin αv chain (particularly preferably, a polypeptide chain consisting of the amino acid sequence shown in SEQ ID NO:1, a polypeptide chain consisting of the partial sequence from Phe at position 31 to Thr at position 1048 of the amino acid sequence shown in SEQ ID NO:1, or a polypeptide chain consisting of the partial sequence from Phe at position 31 to Val at position 992 of the amino acid sequence shown in SEQ ID NO:1), wherein the formed dimer has the ability to bind to an extracellular matrix protein such as laminin or fibronectin to which native integrin αvβ6 or commercially available integrin αvβ6 can bind.

The partial sequence in (VI) as described above includes the partial sequence from Gly at position 22 to Asn at position 707 of the amino acid sequence shown in SEQ ID NO:2. The partial sequences in (VII) and (VIII) as described above include the partial sequence from Gly at position 22 to Cys at position 788 of the amino acid sequence shown in SEQ ID NO:2, and the partial sequence from Gly at position 22 to Asn at position 707 of the amino acid sequence shown in SEQ ID NO:2.

The sequence identity in (VII) as described above is preferably 90% or more, more preferably 95% or more, even more preferably 96% or more, particularly preferably 97% or more, and most preferably 98% or more or 99% or more.

In (VIII) as described above, the term "one or more" means, for example 1 to 100, preferably 1 to 50, preferably 1 to 30, preferably 1 to 20, preferably 1 to 15, preferably 1 to 10, preferably 1 to 5, preferably 1 to 4, preferably 1 to 3, preferably 1 to 2, or preferably 1.

In (III) and (VII) as described above, the amino acid sequence identity can be determined using a method well known to those skilled in the art, for example, sequence analysis software. Examples of the sequence analysis software include, but not limited to, blastp program of BLAST algorithm, and fasta program of FASTA algorithm.

### <4. Test method for irAE enteritis>

The first aspect of the test method of the present invention relates to a method of testing for irAE enteritis, the method comprising:
detecting an antibody that immunologically reacts with a fragment or an entirety of integrin αvβ6 in a sample as an indicator of ulcerative colitis-like irAE enteritis.

The second aspect of the test method of the present invention relates to a method for determining whether or not enteritis developed in a patient receiving ICI treatment is ulcerative colitis-like irAE enteritis, the method comprising:
detecting an antibody that immunologically reacts with a fragment or an entirety of integrin αvβ6 in a sample from the patient as an indicator of ulcerative colitis-like irAE enteritis,
wherein when an antibody titer of the antibody that immunologically reacts with a fragment or an entirety of integrin αvβ6 detected in the sample is higher than an antibody titer of the antibody detected in a control sample, the enteritis developed in the patient is determined to be ulcerative colitis-like irAE enteritis.

The third aspect of the test method of the present invention relates to a method for predicting a risk of developing ulcerative colitis-like irAE enteritis in a patient, the method comprising:
detecting an antibody that immunologically reacts with a fragment or an entirety of integrin αvβ6 in a sample from the patient as an indicator of ulcerative colitis-like irAE enteritis,
wherein when an antibody titer of the antibody that immunologically reacts with a fragment or an entirety of integrin αvβ6 detected in the sample is higher than an antibody titer of the antibody detected in a control sample, the patient is determined to be at risk of developing ulcerative colitis-like irAE enteritis.

Hereinafter, the "fragment or entirety of integrin αvβ6" is collectively referred to as "integrin αvβ6." The above-described first, second and third aspects of the test method of the present invention are collectively referred to as the "test method of the present invention."

The first and second aspects of the test method of the present invention were completed based on the surprising finding that the presence of antibodies (autoantibodies) against integrin αvβ6 in a sample obtained from a patient is indicative of the patient being suffering from ulcerative colitis-like irAE enteritis.

The first and third aspects of the test method of the present invention were completed based on the surprising finding that the presence of antibodies (autoantibodies) against integrin αvβ6 in a sample obtained from a patient is indicative for the patient being at risk of developing ulcerative colitis-like irAE enteritis. According to the present invention, surprisingly, it was shown that patients suffering from ulcerative colitis-like irAE enteritis had expressed antibodies (autoantibodies) against integrin αvβ6 even before receiving ICI treatment.

In the first, second, and third aspects of the test method of the present invention, the irAE enteritis may be "enteritis for which discontinuation of an ICI is recommended, and preferably "ulcerative colitis-like enteritis for which discontinuation of an ICI is recommended".

The sample in the test method of the present invention is a sample derived from a patient. The sample used in the second aspect of the test method of the present invention is a sample from a patient receiving ICI treatment and developing enteritis. The sample used in the third aspect of the test method of the present invention is a sample from a patient before receiving ICI treatment, or a patient receiving ICI treatment and not developing enteritis. In the test method of the present invention, the patient receiving ICI treatment may be a patient receiving combined treatment with an ICI and a cancer chemotherapeutic drug other than an ICI.

As used herein, the expression "developing enteritis" means exhibiting symptoms of enteritis. Examples of the symptoms of enteritis include diarrhea, increased frequency of bowel movements, abdominal pain, mucus stools, and blood in stools.

In the first or second aspects of the test method of the present invention, a sample from a patient exhibiting an ulcerative colitis-like disease condition may be used. In such a case, the test method of the present invention can be used to determine whether the ulcerative colitis-like disease condition is irAE or not. As used herein, the term "ulcerative colitis-like disease condition" refers to a condition exhibiting mucosal findings similar to those of ulcerative colitis, for example, a condition showing diffuse involvement extending proximally from the rectum, with continuous mucosal edema, bleeding, erosion and ulceration. Such disease conditions can be observed by, for example, endoscopy. The formation of crypt abscesses in colon tissues is also included in the "ulcerative colitis-like disease condition". The crypt abscesses can be identified by, for example, removing diseased tissues and observing the diseased tissues under a microscope.

The test method of the present invention comprises a step of detecting an antibody that immunologically reacts with integrin αvβ6 in a sample. As used herein, the term "detecting" refers not only to confirming whether or not the antibody that immunologically reacts with integrin αvβ6 is present in the sample, but also to measuring the amount of the antibody (antibody titer) contained in the sample, i.e., "quantitating" the antibody.

As used herein, the "antibody that immunologically reacts with integrin αvβ6" includes an antibody that specifically binds to integrin αvβ6, and an antibody that specifically binds to integrin αvβ6 to inhibit the binding of integrin αvβ6 to a cell matrix protein such as laminin or fibronectin.

In the test method of the present invention, the detecting may be performed by any method capable of quantitatively or qualitatively detecting the antibody in a sample, which is not particularly limited. For example, an immunological technique can be used for the detecting. Examples of such an immunological technique include Enzyme-Linked ImmunoSorbent Assay (ELISA), immunoprecipitation (IPP), immunoblotting (IB), latex agglutination, immunochromatography, indirect fluorescent antibody method (IF), and radioimmunoassay (RIA). The ELISA method is particularly preferred because a large number of samples can be subjected to the ELIAS method. Specifically, the ELISA method comprises immobilizing integrin αvβ6 as described above, which is an antigen for the antibody, on a solid phase, bringing a sample into contact with the antigen immobilized on the solid phase, and detecting an immune complex between the antigen and the antibody that may be present in the sample by using an enzyme-labeled antibody for detection or the like, and thereby detecting the antibody. A method for suppressing non-specific reactions accompanying the implementation of ELISA, a labeling substance that can be used for the detection, a measurement instrument, and the like are well known in the art and are not particularly limited. As the solid phase for immobilizing the antigen, a solid phase of any shape, such as a plate, bead, or tube, can be used.

For the immunological technique such as ELISA, a step of bringing a sample that may contain the antibody into contact with the antigen, a necessary washing step, a necessary labeling step, and a necessary detection step can be carried out in appropriate buffer solutions, preferably buffer solutions containing one or more metal ions selected from the group consisting of a calcium ion, a magnesium ion, a manganese ion, sodium, lithium, and the like. As the metal ion, a divalent metal ion such as a calcium ion, a magnesium ion, or a manganese ion is preferably selected, and one or two of a calcium ion, a magnesium ion, and a manganese ion are particularly preferably selected. The concentration of the metal ions in the buffer solution is not particularly limited, and the total concentration of metal ions may be, for example, 0.02 mM to 200 mM in total, preferably 0.2 mM to 20 mM, and particularly preferably 0.4 mM to 10 mM. Since the detection sensitivity is increased in a buffer solution containing these metal ions, such a buffer solution is preferable.

In the detection step, the amount of the antibody that immunologically reacts with integrin αvβ6 can be determined, for example, by measuring the amount of the label of the antibody for detection bound to the immune complex between the antibody and the antigen. In addition, the amount of the antibody in a sample to be tested can be calculated from the measured amount of the label based on a calibration curve that is prepared by using a positive standard solution containing a known concentration of the antibody.

In the detection step, a determination of whether the antibody that immunologically reacts with integrin αvβ6 is present or not (a determination of positive/negative) can be conducted by comparing the measurement value of the antibody in a sample obtained from a patient with the measurement value of the antibody in a sample obtained from a control. The measurement of the antibody in a sample obtained from a control may be performed simultaneously with the measurement of the antibody in a sample obtained from a patient, or may be performed in advance. When the measurement value of the antibody in a sample obtained from a patient is greater, preferably significantly greater, than the measurement value of the antibody in a sample obtained from a control, the patient can be determined to be positive.

In the detection step, the antibody titer of the antibody in the control sample to be compared with the antibody titer of the antibody in the patient-derived sample may be measured simultaneously with measurement of the antibody in the patient-derived sample, or may be measured in advance.

As used herein, the "control" may be an animal of the same species as the patient or an animal of different species from the patient, and is preferably an animal of the same species as the patient. Additionally, the "control" is an animal not suffering from ulcerative colitis-like irAE enteritis, for example, an animal not exhibiting ulcerative colitis-like disease conditions, an animal not receiving ICI treatment, or an animal not suffering from cancer. The animal not suffering from ulcerative colitis-like irAE enteritis includes an animal that has never suffered from ulcerative colitis-like irAE enteritis, and an animal that has suffered from ulcerative colitis-like irAE enteritis in the past and has now recovered, for example, an animal that has been cured for more than several years, preferably more than ten years. The animal not receiving ICI treatment includes an animal that has never received ICI treatment, and an animal that has received ICI treatment in the past and is not currently receiving said treatment, for example, an animal that has not received ICI treatment for more than several years, preferably more than ten years, since the animal received the last ICI administration. The animal not suffering from cancer includes an animal that has never suffered from cancer, and an animal that has suffered from cancer in the past and has now recovered, for example, an animal that has been cured for more than several years, preferably more than ten years. The control is preferably an animal not exhibiting ulcerative colitis-like disease conditions and not suffering from cancer, more preferably a healthy animal that does not exhibit any disease conditions and has not been diagnosed as suffering from or suspected of suffering from any diseases, and even more preferably a healthy subject.

The isotype of the antibody that immunologically reacts with integrin αvβ6 to be detected in the detection step of the test method of the present invention is not particularly limited. The isotype may be an IgG antibody, an IgA antibody, an IgM antibody, or an IgE antibody, and an IgG antibody or an IgA antibody is particularly preferred. It is particularly preferable to detect IgG or IgA as the antibody that immunologically reacts with integrin αvβ6. The subclass of an IgG antibody is not particularly limited, and for example, an IgG1 antibody, an IgG2 antibody, an IgG3 antibody, or an IgG4 antibody may be detected. As the IgG antibody that immunologically reacts with integrin αvβ6, an IgG1 antibody, an IgG2 antibody, or an IgG4 antibody is more preferably detected, and an IgG1 antibody is most preferably detected.

According to the test method of the present invention, it is possible to test for the presence or absence of ulcerative colitis-like irAE enteritis in a patient, or the risk of developing ulcerative colitis-like irAE enteritis in a patient, or the presence or absence of "enteritis for which discontinuation of an ICI is recommended" in a patient, or the risk of developing "enteritis for which discontinuation of an ICI is recommended" in a patient. Thus, a therapeutic strategy for the patient can be decided based on a result of the test. For example, when the enteritis developed in the patient is determined to be ulcerative colitis-like irAE enteritis or "enteritis for which discontinuation of an ICI is recommended" based on a test result of the second aspect of the test method of the present invention, it is preferable to discontinue the administration of an ICI to the patient and/or administer an immunosuppressant to the patient. For example, when the patient is determined to be at risk of developing ulcerative colitis-like irAE enteritis or at risk of developing "enteritis for which discontinuation of an ICI is recommended" based on a test result of the third aspect of the test method of the present invention, it is preferable to administer treatment other than ICI treatment in the case of the patient prior to ICI treatment, discontinue the administration of an ICI in the case of the patient receiving ICI treatment, and/or administer an immunosuppressant to the patient.

A cutoff value for the determination based on test results of the second or third aspect of the test method of the present invention can be appropriately determined by a person skilled in the art using a method known in the art. For example, the cutoff value may be calculated by adding a value three times as much as a standard deviation (SD) to the average value of test results (antibody titer) in the control group.

According to the test method of the present invention, in particular, severe ulcerative colitis-like irAE enteritis can be detected. As used herein, the term "severe" may mean, for example, enteritis of grade 3 or higher based on the Common Terminology Criteria for Adverse Events (CTCAE) version 5.0 published by the Cancer Therapy Evaluation Program (CTEP) of the National Cancer Institute (NCI) in the United States in November 2017, or enteritis of Mayo score 11 or higher as the sum of scores in four categories, or enteritis of score 8 or higher as the sum of scores in three categories (Partial Mayo score). Regarding the CTCAE, "grade" indicates the severity of adverse events, and grade 1 is defined as being "mild", grade 2 is defined as being "moderate", grade 3 is defined as being "severe", grade 4 is defined as being "life-threatening", and grade 5 is defined as being "death". The Mayo score is an established assessment tool for determining the severity of ulcerative colitis in clinical tests, and the assessment is made based on the sum of scores in four categories: stool frequency (normal frequency: 0 points, 1-2 stools/day more than normal: 1 point, 3-4 stools/day more than normal: 2 points, 5 stools/day more than normal: 3 points), rectal bleeding (none: 0 points, visible blood with stool less than half the time: 1 point, visible blood with stool half of the time or more: 2 points, passing blood alone: 3 points), mucosal appearance at endoscopy (normal or inactive disease: 0 points, mild disease: 1 point, moderate disease: 2 points, severe disease: 3 points), and physician rating of disease activity (normal: 0 points, mild: 1 point, moderate: 2 points, severe: 3 points). A total Mayo score of 3 to 5 is considered "mild", a total Mayo score of 6 to 10 is considered "moderate", and a total Mayo score of 11 to 12 is considered "severe". Regarding the Partial Mayo score, the assessment is made based on the sum of scores in three categories: stool frequency, rectal bleeding, and physician rating of disease activity. A total Partial Mayo score of 2 to 4 is considered "mild", a total Partial Mayo score of 5 to 7 is considered "moderate", and a total Partial Mayo score of 8 to 9 is considered "severe". According to the present invention, it was found that many of anti-integrin αvβ6 antibody-positive patients suffer from severe enteritis.

### <5. Test kit or test reagent for irAE enteritis>

The first aspect of the test kit or test reagent of the present invention relates to a test kit or test reagent for testing for irAE enteritis, comprising a fragment or an entirety of integrin αvβ6.

The second aspect of the test kit or test reagent of the present invention relates to a test kit or test reagent for determining whether enteritis developed in a patient receiving ICI treatment is ulcerative colitis-like irAE enteritis or not, comprising a fragment or an entirety of integrin αvβ6.

The third aspect of the test kit or test reagent of the present invention relates to a test kit or test reagent for predicting the risk of developing ulcerative colitis-like irAE enteritis in a patient receiving ICI treatment, comprising a fragment or an entirety of integrin αvβ6.

Hereinafter, the first, second, and third aspects if the test kit or test reagents of the present invention are collectively referred to as "the test kit or test reagent of the present invention".

In the first, second, and third aspects of the test kit or test reagent of the present invention, the irAE enteritis may be "enteritis for which discontinuation of an ICI is recommended", and preferably "ulcerative colitis-like enteritis for which discontinuation of an ICI is recommended".

The test kit or test reagent of the present invention can be used in the test method of the present invention.

The test kit of the present invention may contain reagents necessary for immunological measurement, for example, a buffer, and one or more metal salts selected from the group consisting of a calcium salt, a magnesium salt, a manganese salt, a sodium salt, a lithium salt, etc., as necessary.

The test reagent of the present invention may be a liquid or solid composition containing integrin αvβ6 and a carrier acceptable for the purpose of formulation, such as a solvent, an excipient, etc.

In the test kit or test reagent of the present invention, the form of integrin αvβ6 is not particularly limited, and integrin αvβ6 may be in a solution state, in a dry state, or immobilized on a solid phase. In the case of integrin αvβ6 in a dry state, the test kit or test reagent of the present invention may comprise a buffer or a solvent for moving the integrin αvβ6 from a dry state to a solution state before use.

The test kit or test reagent comprising integrin αvβ6 immobilized on a solid phase can be used in ELISA. The solid phase may be in any form, for example, a plate, beads, or a tube. The test kit for use in ELISA can comprise, in addition to the solid phase, a positive standard solution, a negative standard solution, a blocking solution, a washing solution, a diluent for sample, an antibody for detection, a substrate, and the like. The antibody for detection may be labeled with a detectable label, such as an enzyme. As the positive standard solution, a diluted solution of serum from a patient with ulcerative colitis-like irAE enteritis containing an autoantibody to integrin αvβ6, or a solution containing an antibody to integrin αvβ6 is used. The antibody added to the solution is preferably of the same isotype as that of the autoantibody to be measured, such as an IgG antibody, an IgA antibody, an IgM antibody, an IgE antibody, or the like. The negative standard solution includes a diluted solution of serum from an animal (e.g., human) not suffering from ulcerative colitis-like irAE enteritis, a serum from a patient with another intestinal disease that is medically distinct from ulcerative colitis-like irAE enteritis, a serum from a healthy subject (control serum), and dilutions thereof.

### <6. Method of treating ulcerative colitis-like irAE enteritis>

The first aspect of the treatment method of the present invention relates to a method of treating ulcerative colitis-like irAE enteritis, the method comprising:
detecting an antibody that immunologically reacts with a fragment or an entirety of integrin αvβ6 in a sample from a patient receiving ICI treatment and developing enteritis, and
discontinuing ICI treatment and/or administering an immunosuppressant to the patient when an antibody titer of the antibody detected in a sample from the patient is higher than an antibody titer of the antibody detected in a control sample.

The second aspect of the treatment method of the present invention relates to a method of treating enteritis developed in a patient receiving combined treatment with an ICI and a cancer chemotherapeutic drug other than an ICI, the method comprising:
detecting an antibody that immunologically reacts with a fragment or an entirety of integrin αvβ6 in a sample from the patient, and
discontinuing the ICI in the patient and/or administering an immunosuppressant to the patient when an antibody titer of the antibody detected in the sample from the patient is higher than an antibody titer of the antibody detected in a control sample, or
discontinuing the cancer chemotherapeutic drug other than an ICI in the patient when an antibody titer of the antibody detected in the sample from the patient is equivalent to or lower than an antibody titer of the antibody detected in a control sample or when the antibody is not detected in the sample from the patient.

Hereinafter, the first and second aspects of the treatment method of the present invention are collectively referred to as "the treatment method of the present invention".

In the first aspect of the treatment method of the present invention, the ulcerative colitis-like irAE enteritis may be "ulcerative colitis-like enteritis for which discontinuation of an ICI is recommended".

In the treatment method of the present invention, preferably, the ICI treatment is discontinued and an immunosuppressant is administered to the patient when an antibody titer of the antibody detected in a sample from the patient is higher than an antibody titer of the antibody detected in a control sample.

The "immunosuppressant" used in the treatment method of the present invention is not limited as long as it has the effect of suppressing an inflammatory response accompanying excessive immune responses occurring in the body. Examples of the immunosuppressant include steroids (e.g., prednisolone, rinderon, decadron, saxizon, medrol, etc.), anti-TNFα antibodies (e.g., infliximab, adalimumab, golimumab, etc.), tacrolimus, cyclosporine, and mycophenolate mofetil. Two or more types of immunosuppressants may be used.

As described above, the antibody that immunologically reacts with integrin αvβ6 in a sample is useful as an indicator of ulcerative colitis-like irAE enteritis. Thus, when an antibody titer of the antibody detected in a sample from a patient is higher than an antibody titer of the antibody detected in a control sample, the treatment for irAE, i.e., discontinuation of an ICI and/or administration of an immunosuppressant is given to the patient. When the antibody is not detected in a sample from a patient or when an antibody titer of the antibody detected in a sample from a patient is equivalent to or lower than an antibody titer of the antibody detected in a control sample, the treatment for general enteritis (i.e., non-irAE enteritis) is administered to the patient. Examples of the treatment for general enteritis include administration of an antiflatulent, and fasting if necessary, or administration of an antibiotic when bacterial infection is suspected. The treatment for general enteritis may be appropriately determined by a person skilled in the art based on the symptoms of enteritis or the progress of treatment. For example, when no recovery is observed, an ICI is discontinued and a low dose of an immunosuppressant may be administered empirically.

In the treatment method of the present invention, the step of detecting the antibody and the method of comparing the antibody titers are the same as those explained for the test method of the present invention.

### <7. Method of evaluating efficacy in treatment of ulcerative colitis-like irAE enteritis>

The first aspect of the evaluation method of therapeutic effect of the present invention relates to a method of evaluating efficacy in treatment of ulcerative colitis-like irAE enteritis, the method comprising:
detecting an antibody that immunologically reacts with a fragment or an entirety of integrin αvβ6 in a sample obtained from a patient receiving the treatment of ulcerative colitis-like irAE enteritis.

As described above, the antibody that immunologically reacts with integrin αvβ6 in a sample is useful as an indicator of ulcerative colitis-like irAE enteritis. Thus, the amount of the antibody in a sample obtained from a patient receiving treatment for ulcerative colitis-like irAE enteritis decreases when the treatment is effective, and remains unchanged from before the start of treatment or increases when the treatment is not fully effective. Therefore, the efficacy of treatment can be evaluated by detecting the antibody in a sample obtained from a patient receiving the treatment for ulcerative colitis-like irAE enteritis.

When the quantitative result of the antibody in a sample obtained from a patient receiving treatment for ulcerative colitis-like irAE enteritis, obtained in the detection step of the evaluation method of therapeutic effect of the present invention is smaller than, for example, the quantitative result of the antibody in a sample obtained from the same individual before starting the treatment, the treatment can be evaluated as being effective. On the other hand, when the former is equivalent to or greater than the latter, the treatment can be evaluated as being ineffective or insufficient. Based on such evaluation results, continuation of the treatment, change of the treatment plan, or discontinuation of the treatment can be decided.

When the quantitative result of the antibody in a sample obtained from a patient receiving treatment for ulcerative colitis-like irAE enteritis, obtained in the detection step of the evaluation method of therapeutic effect of the present invention is equivalent to or smaller than, for example, the quantitative result of the antibody in a sample obtained from a healthy individual of the same species as the patient, the treatment can be evaluated as being effective. On the other hand, when the former is greater than the latter, the treatment can be evaluated as being ineffective or insufficient. Based on such evaluation results, continuation of the treatment, change of the treatment plan, or discontinuation of the treatment can be decided.

In the evaluation method of the present invention, the step of detecting the antibody and the like are the same as those explained for the test method of the present invention.

In the evaluation method of the present invention, the ulcerative colitis-like irAE enteritis may be "ulcerative colitis-like enteritis for which discontinuation of an ICI is recommended".

### <8. Method of screening for a candidate substance of a therapeutic agent for ulcerative colitis-like irAE enteritis>

The first aspect of the screening method of the present invention relates to a method of screening for a candidate substance for treating ulcerative colitis-like irAE enteritis, the method comprising:
detecting an antibody that immunologically reacts with a fragment or an entirety of integrin αvβ6 in a sample obtained from an animal treated with a test substance, and
selecting the test substance as a candidate substance for treating ulcerative colitis-like irAE enteritis when the antibody in the sample is reduced by treating the animal with the test substance.

The animal is an animal, preferably a mammal, more preferably a non-human mammal, suffering from ulcerative colitis-like irAE enteritis.

As described above, the antibody that immunologically reacts with integrin αvβ6 in a sample is useful as an indicator of ulcerative colitis-like irAE enteritis. Thus, a test substance can be selected as a candidate substance of a therapeutic agent for ulcerative colitis-like irAE enteritis when the antibody in the sample is reduced by treatment with the test substance.

In the screening method of the present invention, the step of detecting the antibody, and the like are the same as those explained for the test method of the present invention.

In the screening method of the present invention, the test substance refers to a test substance that may be a candidate for a new drug, and is not particularly limited and is.

In the selection step of the screening method of the present invention, when the quantitative result of the antibody in a sample obtained from an animal treated with a test substance, obtained by the antibody detection step is smaller than, for example, the quantitative result of the antibody in a sample obtained from the same individual before treatment with the test substance, it is decided that the antibody in the sample is reduced by the treatment with the test substance, and thus the test substance can be selected as a candidate substance of a therapeutic agent for ulcerative colitis-like irAE enteritis.

In the selection step of the screening method of the present invention, when the quantitative result of the antibody in a sample obtained from an animal treated with a test substance, obtained by the antibody detection step is equivalent to or smaller than, for example, the quantitative result of the antibody in a sample obtained from a healthy individual of the same species as the animal, it is decided that the antibody in the sample is reduced by the treatment with the test substance, and thus the test substance can be selected as a candidate substance of a therapeutic agent for ulcerative colitis-like irAE enteritis.

In the screening method of the present invention, the ulcerative colitis-like irAE enteritis may be "ulcerative colitis-like enteritis for which discontinuation of an ICI is recommended".

### EXAMPLES

Hereinafter, the present invention is explained in more detail using Examples which the present invention is not limited to.

### <Example 1: Diagnosis of ulcerative colitis-like irAE enteritis based on anti-integrin αvβ6 antibody - 1>

Human integrin αvβ6 (3817-AV, R&D system) was immobilized on a solid phase as an antigen. The presence of antibodies that bind to the antigen was determined by ELISA in the sera of patients with ulcerative colitis-like irAE enteritis and patients with non-ulcerative colitis-like irAE enteritis. In the ELISA, the autoantibodies to be detected were human IgG, and a rabbit anti-human IgG polyclonal antibody labeled with horseradish peroxidase (HRP) was used as an antibody for detection to detect the antibodies in the sera of the patients that bound to the antigen immobilized on the solid phase.

### 1. Serum samples

Serum samples were obtained from nine patients that were diagnosed as having ulcerative colitis-like irAE enteritis by endoscopy. As controls, serum samples were obtained from 13 patients diagnosed as having non-ulcerative colitis-like irAE enteritis by endoscopy.

### 2. Procedure

Unless otherwise specified, ELISA Starter Accessory Kit (E101, Bethyl Laboratories) was used in experiments as described below. An ELISA coating buffer, an ELISA wash solution, an ELISA blocking buffer, and a conjugate diluent were prepared according to instructions attached to the kit, except that CaCl₂ and MgCl₂ were added to the ELISA wash solution, ELISA blocking buffer and conjugate diluent at a final concentration of 1 mM. All steps were performed at room temperature unless otherwise stated.

### 2.1. Coating with antigen

(1) The antigen was diluted with the ELISA coating buffer of the kit to prepare a solution containing 2 µg/ml of the antigen. Then, 100 µl of this solution was added into each well of a microwell plate of the kit.
(2) The microwell plate was incubated at 4°C for 60 minutes.
(3) After incubation, the solution was removed from each well by aspiration.
(4) Each well was washed with the ELISA wash solution of the kit. Specifically, each well was filled with the ELISA wash solution, and then the ELISA wash solution was removed by aspiration. This procedure was repeated three times.

### 2.2 Blocking

(1) To each well of the microwell plate, 200 µl of the ELISA blocking buffer of the kit was added.
(2) The plate was incubated for 30 minutes.
(3) After incubation, the ELISA blocking buffer was removed, and each well was washed three times.

### 2.3 Test Samples

(1) The serum samples from patients with ulcerative colitis-like irAE enteritis (n=9) or the serum samples from patients with non-ulcerative colitis-like irAE enteritis (n=13) were diluted at a ratio of 1:100 with the conjugate diluent of the kit.
(2) To each well of the blocked microwell plate, 100 µl of the serum diluted solution obtained by the above-described dilution was added.
(3) The plate was incubated for 60 minutes.
(4) After incubation, the serum diluted solution was removed, and each well was washed five times.

### 2.4 HRP-conjugated antibody for detection

(1) The antibody for detection conjugated with HRP (abcam6759, rabbit anti-human IgG H&L (HRP)) was diluted with the conjugate diluent of the kit at a ratio of 1:50,000.
(2) To each well of the microwell plate contacted with the serum, 100 µl of the diluted solution of antibody for detection obtained by the above-descried dilution was added.
(3) The plate was incubated for 60 minutes.
(4) After incubation, the diluted solution of antibody for detection was removed, and each well was washed five times.

### 2.5 Enzyme-substrate reaction

(1) A TMB (3,3',5,5'-tetramethylbenzidine) solution was prepared according to the manufacturer's recommended conditions.
(2) To each well of the microwell plate contacted with the antibody for detection, 100 µl of the TMB solution was added.
(3) The plate was incubated for 7 to 8 minutes.
(4) To each well, 100 µl of 0.18 M H₂SO₄ was added to stop oxidation of TMB.
(5) A color produced by the oxidation reaction was measured at a wavelength of 450 nm using a microplate reader.

### 3. Results and Discussion

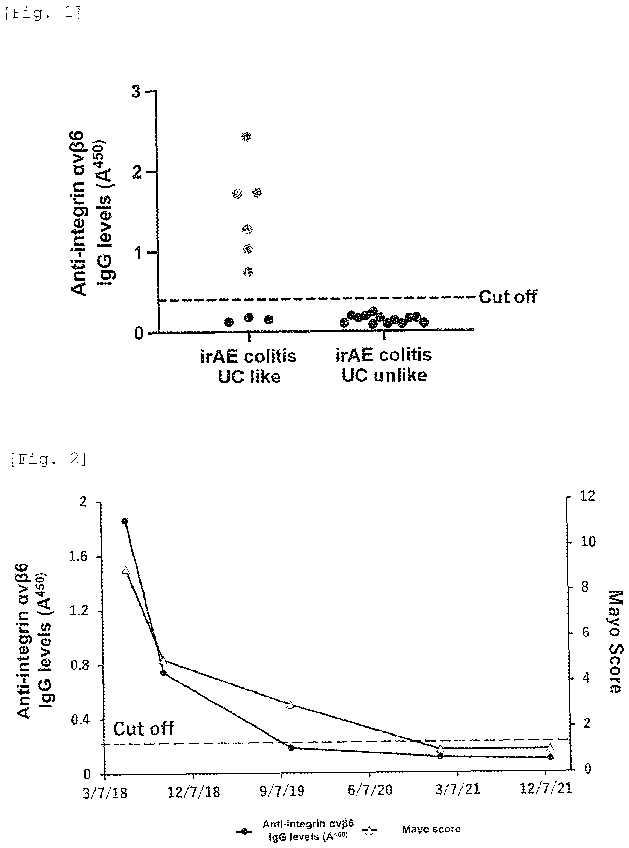

Results are shown in Figure 1. In Figure 1, the vertical axis indicates absorbance (OD value) at 450 nm, which is proportional to the amount of the antibody for detection on the solid phase. The "UC-like irAE colitis" indicates the serum samples from patients with ulcerative colitis-like irAE enteritis, and the "UC-unlike irAE colitis" indicates the serum samples from patients with non-ulcerative colitis-like irAE enteritis (control). The cutoff value, indicated by a dashed line, is a value calculated by adding a value three times as much as a standard deviation (SD) to the mean absorbance of the control serum samples. Serum samples from healthy individuals were used as controls.

Six out of nine serum samples from patients with ulcerative colitis-like irAE enteritis were positive for anti-human integrin αvβ6 antibody, and thus sensitivity was 66.6%. On the other hand, 13 out of 13 serum samples from patients with non-ulcerative colitis-like irAE enteritis were negative for anti-human integrin αvβ6 antibody, and thus specificity was 100% (Figure 1). The sensitivity and specificity were calculated using the following formulae. Sensitivity (%) = [number of patients judged to be positive for ulcerative colitis-like irAE enteritis based on antibody titer/total number of patients with ulcerative colitis-like irAE enteritis tested] x 100 Specificity (%) = [number of patients judged to be negative for ulcerative colitis-like irAE enteritis based on antibody titer/total number of patients with non-ulcerative colitis-like irAE enteritis tested] x 100

These results support that an increase in the serum level of autoantibodies against human integrin αvβ6 is a diagnostic indicator for ulcerative colitis-like irAE enteritis, and that ulcerative colitis-like irAE enteritis can be diagnosed based on the blood level of autoantibodies against human integrin αvβ6.

### <Example 2: Correlation between anti-integrin αvβ6 antibody titer and clinical presentation - 1>

In order to confirm that there was a correlation between anti-integrin αvβ6 antibody titer and clinical presentation, in a patient diagnosed with ulcerative colitis-like irAE enteritis, the serum levels of anti-integrin αvβ6 antibody were measured and clinical images of the intestines were taken over time.

The patient was a 57-year-old female with peritoneal cancer who underwent ICI treatment with atezolizumab from October 12, 2017 to January 23, 2018, and developed ulcerative colitis-like irAE enteritis on February 27, 2018. After the development of ulcerative colitis-like irAE enteritis, atezolizumab was discontinued, and however, there was no improvement. The patient started to have oral treatment with 30 mg/day of prednisolone from May 29, 2018. The dose of prednisolone was gradually reduced, and the treatment with prednisolone was discontinued on October 1, 2018. Serum samples were obtained from the patient on May 7, 2018, September 10, 2018, October 7, 2019, December 7, 2020, and December 13, 2021. The serum samples were subjected to measurement of anti-integrin αvβ6 antibody titer by ELISA as described in Example 1. In addition, lower gastrointestinal endoscopy was performed on April 27, 2018, September 7, 2018, and October 7, 2019. In addition, the patient was evaluated using Mayo score based on findings over the three days before and after May 7, 2018, September 10, 2018, October 7, 2019, December 7, 2020, and December 13, 2021. The Mayo score consists of four categories: stool frequency, rectal bleeding, mucosal appearance at endoscopy, and physician rating of disease activity (Table 1). The mucosal appearance at endoscopy was assessed using data from one day, and the remaining three categories were assessed based on data from three days. The assessment by the Mayo score is made based on the sum of scores in four categories, and a higher score indicates more severe symptoms.

**[Table 1]**

| Mayo score | |
|---|---|
| 1. Stool frequency | 0: normal frequency |
| | 1: 1-2 stools/day more than normal |
| | 2: 3-4 stools/day more than normal |
| | 3: ≥5 stools/day more than normal |
| 2. Rectal bleeding | 0: none |
| | 1: visible blood with stool less than half the time |
| | 2: visible blood with stool half of the time or more |
| | 3: passing blood alone: |
| 3. Mucosal appearance at endoscopy | 0: normal or inactive disease |
| | 1: mild disease |
| | 2: moderate disease |
| | 3: severe disease |
| 4. Physician rating of disease activity | 0: normal |
| | 1: mild |
| | 2: moderate |
| | 3: severe |

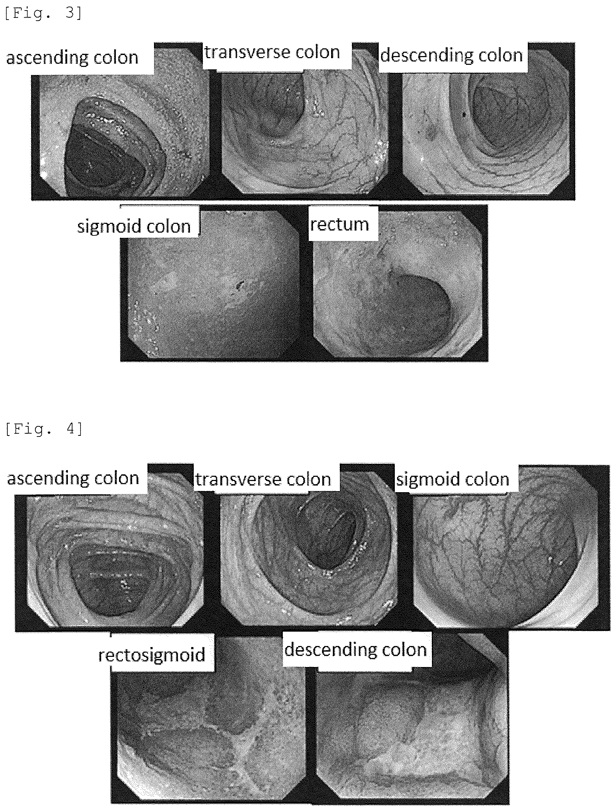

Results are shown in Figures 2 to 5. Figure 2 shows the changes over time of the antibody titer in the patient serum samples and the Mayo score, an assessment tool for determining the severity of ulcerative colitis. In Figure 2, the left vertical axis indicates absorbance at 450 nm, which is proportional to the amount of the antibody for detection on the solid phase, the right vertical axis indicates the Mayo score, and the horizontal axis indicates the date. In Figure 2, the cutoff value of the antibody titer shown by a dashed line was calculated by using the average value measured in a group of healthy subjects plus 3SD. Figures 3, 4, and 5 are lower gastrointestinal endoscopic photographs taken on April 27, 2018, September 7, 2018, and October 7, 2019, respectively.

The anti-integrin αvβ6 antibody titers in the patient serum samples were 1.86 on May 7, 2018, 0.74 on September 10, 2018, and 0.18 on October 7, 2019. As is clear from Figure 2, the antibody titer was high at the time of the onset of ulcerative colitis-like irAE enteritis, and the antibody titer decreased after discontinuation of the ICI and start of steroid treatment. Furthermore, as is clear from Figures 3, 4, and 5, colonoscopic images at the time of high antibody titer showed inflammatory appearance including erythema, erosion, and ulceration from the ascending colon to the sigmoid colon and rectum, and the inflammatory appearance was improved as the antibody titer decreased, and when the antibody titer fell below the cutoff value, the inflammatory appearance was hardly observed. In addition, the Mayo Score also improved as the antibody titer decreased. Thus, it was demonstrated that the anti-integrin αvβ6 antibody titer correlates with clinical presentation. Therefore, the anti-integrin αvβ6 antibody titer can be used as an indicator of the degree of progression of ulcerative colitis-like irAE enteritis or the severity of ulcerative colitis-like irAE enteritis. Further, it was also found that the therapeutic effect for ulcerative colitis-like irAE enteritis can be evaluated by observing the changes in the anti-integrin αvβ6 antibody titer over time.

### <Example 3: Correlation between anti-integrin αvβ6 antibody titer and clinical presentation - 2>

An 82-year-old male patient with non-small cell lung cancer received ICI treatment with pembrolizumab from October 2021 and developed ulcerative colitis-like irAE enteritis on February 28, 2022. From that day, pembrolizumab was discontinued, and the symptoms improved naturally. Serum samples were obtained from the patient on April 11, 2022, April 25, 2022, and July 21, 2022. The serum samples were subjected to measurement of anti-integrin αvβ6 antibody titer by ELISA as described in Example 1. In addition, lower gastrointestinal endoscopy was performed on March 30, 2022. In addition, the patient was assessed using Partial Mayo score based on findings over the three days before and after April 11, 2022, April 25, 2022, and July 21, 2022. The Partial Mayo score consists of three categories excluding mucosal appearance at endoscopy: stool frequency, rectal bleeding, and physician rating of disease activity (Table 1).

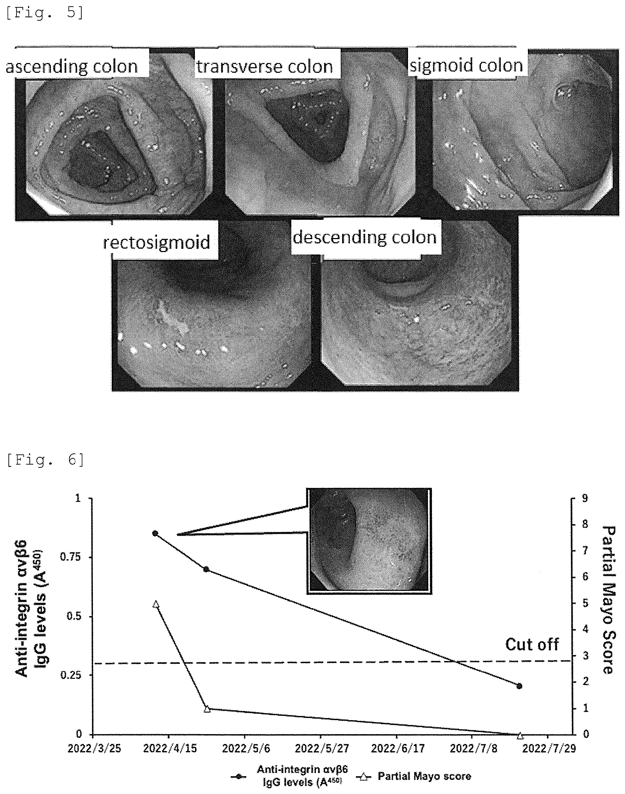

Results are shown in Figure 6. Graphs in Figure 6 show changes over time in antibody titer in the patient's serum samples and Partial Mayo score. The left vertical axis indicates absorbance at 450 nm, which is proportional to the amount of the antibody for detection on the solid phase. The right vertical axis indicates Partial Mayo score. The horizontal axis indicates the date. In Figure 6, the cutoff value of the antibody titer shown by a dashed line was calculated by using the average value measured in a group of healthy subjects plus 3SD.

As is clear from Figure 6, during development of irAE enteritis, the endoscopic image showed inflammatory appearance in the rectum and the antibody titer was as high as 0.85. The antibody titer was subsequently decreased as the symptoms improved. Although the endoscopic examination was performed only one time, the Partial Mayo score excluding endoscopic findings showed improvement tendency.

### <Example 4: Anti-integrin αvβ6 antibody titer before onset of ulcerative colitis-like irAE enteritis>

In a cancer patient, the anti-integrin αvβ6 antibody titer in blood was measured before the start of ICI treatment, after the start of ICI treatment, and after the onset of ulcerative colitis-like irAE enteritis. The antibody titer was measured in the same manner as in Example 1.

The patient was a 69-year-old female with malignant melanoma. Although the patient had resection surgery in May 2018, a recurrence was found in December 2019. The patient had second surgery in January 2020. The patient began to receive ICI treatment from March 2020. The patient received administration of nivolumab in March 2020, and administration of nivolumab and ipilimumab on June 8, 2020. On June 15, 2020, it was found that the patient developed ulcerative colitis-like irAE enteritis by endoscopy. Serum samples were obtained from the patient on February 17, 2020 before the start of ICI treatment, on June 1, 2020 after the start of ICI treatment and before the onset of irAE, and on June 29, July 13, and July 20, 2020 after the onset of irAE. The serum samples were subjected to measurement of anti-integrin αvβ6 antibody titers.

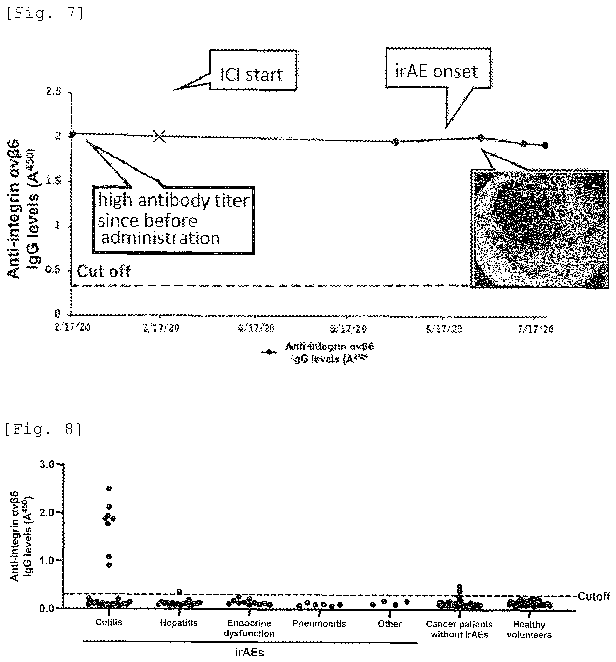

Results are shown in Figure 7. A graph in Figure 7 shows changes over time in antibody titer in the patient's serum samples. The vertical axis indicates absorbance at 450 nm, which is proportional to the amount of the antibody for detection on the solid phase. The horizontal axis indicates the date. Points in the graph indicate the dates at which the serum samples were collected: February 17, 2020, June 1, 2020, June 29, 2020, July 13, 2020, and July 20, 2020. The endoscopic image in Figure 7 was taken when the onset of ulcerative colitis-like irAE enteritis was found (June 15, 2020). In Figure 7, the cutoff value of the antibody titer shown by a dashed line was calculated by using the average value measured in a group of healthy subjects plus 3SD.

As is clear from Figure 7, it was found that patients who will develop ulcerative colitis-like irAE enteritis in the future have a high antibody titer of anti-integrin αvβ6 antibody even before the start of ICI treatment. Therefore, the anti-integrin αvβ6 antibody is useful as an indicator of a risk of developing ulcerative colitis-like irAE enteritis in the future, before the start of ICI treatment or before the onset of enteritis.

### <Example 5: Diagnosis of ulcerative colitis-like irAE enteritis based on anti-integrin αvβ6 antibody - 2>

### (1) Antibody test

Human integrin αvβ6 (3817-AV, R&D system) was immobilized on a solid phase as an antigen. The presence of antibodies that bind to the antigen in the sera of patients with irAE enteritis was determined by ELISA as described in Example 1. As controls, serum samples from other irAE patients (hepatitis, endocrine dysfunction, pneumonitis, and others), cancer patients without irAE, and healthy subjects were tested in the same way.

Results are shown in Figure 8. In Figure 8, the vertical axis indicates absorbance at 450 nm, which is proportional to the amount of the antibody for detection on the solid phase. The cutoff value shown by a dashed line is a value calculated by adding a value three times as much as a standard deviation (SD) to the mean absorbance of the serum samples from healthy subjects.

Eight out of 26 serum samples from patients with irAE enteritis were positive for anti-human integrin αvβ6 antibody, and thus sensitivity was 30.8%. On the other hand, 154 out of 157 control serum samples were negative for anti-human integrin αvβ6 antibody, and thus specificity was 98.1% (Figure 8). The sensitivity and specificity were calculated using the following formulae. Sensitivity (%) = [number of patients judged to be positive for irAE enteritis based on antibody titer/total number of patients with irAE enteritis tested] x 100 Specificity (%) = [number of patients judged to be negative for irAE enteritis based on antibody titer/total number of patients with non-irAE enteritis tested] x 100

### (2) Endoscopic imaging examination - 1

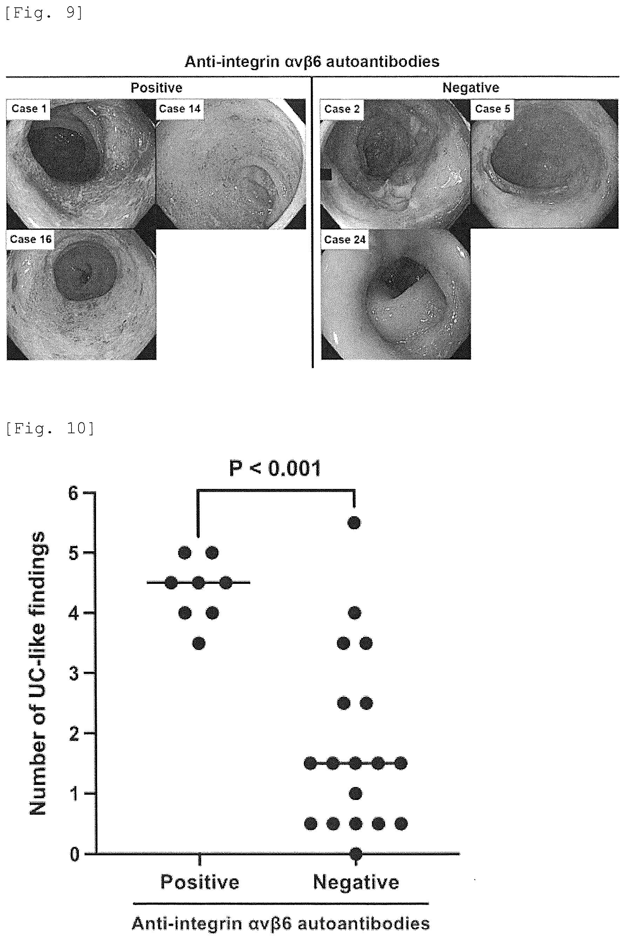

Furthermore, endoscopic images of patients with irAE enteritis were examined. As a result, the antibody-positive patients had many endoscopic findings similar to ulcerative colitis, including diffuse or continuous erythema, granular mucosa, and loss of vascular pattern. On the other hand, the antibody-negative patients scarcely had endoscopic findings similar to ulcerative colitis (Figure 9).

### (3) Endoscopic imaging examination - 2

Next, in order to make more objective evaluation, two IBD specialists, blinded to the antibody titers, evaluated how many characteristics of the six characteristics of ulcerative colitis listed below the irAE enteritis patients had. Evaluation results by the two specialists were averaged.
1. Diffuse or continuous erythema
2. Continuous disease from rectum
3. Granularity
4. Loss of vascular pattern
5. Bleeding
6. Ulceration

Results are shown in Figure 10. All of the eight antibody-positive patients had three or more characteristic findings of ulcerative colitis. On the other hand, only four out of the 18 antibody-negative patients showed three or more characteristic findings of ulcerative colitis. Therefore, it was demonstrated that the antibody-positive patients had more findings characteristic findings of ulcerative colitis with a significant difference as compared to the antibody-negative patients.

These results support that an increase in the serum level of autoantibodies against human integrin αvβ6 is a diagnostic indicator for ulcerative colitis-like irAE enteritis, and that ulcerative colitis-like irAE enteritis can be diagnosed based on the blood level of autoantibodies against human integrin αvβ6.

### <Example 6: Correlation between anti-integrin αvβ6 antibody titer and clinical presentation - 3>

A 69-year-old male patient with renal cancer received ICI treatment with pembrolizumab from March 18, 2019, and developed ulcerative colitis-like irAE enteritis on November 1, 2020. The antibody titer decreased by discontinuation of the ICI and treatment with a steroid. Serum samples were obtained from the patient on December 16, 2020 and September 15, 2022. The serum samples were subjected to measurement of anti-integrin αvβ6 antibody titer by ELISA as described in Example 1. The patient was assessed using Partial Mayo score. The Partial Mayo score consists of three categories excluding mucosal appearance at endoscopy: stool frequency, rectal bleeding, and physician rating of disease activity (Table 1).

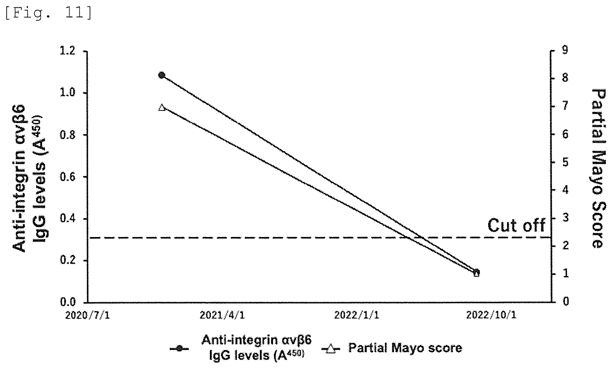

Results are shown in Figure 11. Graphs in Figure 11 show changes over time of the antibody titer in the patient's serum samples and Partial Mayo score. The left vertical axis indicates absorbance at 450 nm, which is proportional to the amount of the antibody for detection on the solid phase. The right vertical axis indicates Partial Mayo score. The horizontal axis indicates the date. In Figure 11, the cutoff value of the antibody titer shown by a dashed line was calculated by using the average value measured in a group of healthy subjects plus 3SD.

As is clear from Figure 11, during the development of irAE enteritis, the endoscopic image showed inflammatory appearance in the rectum and the antibody titer was as high as 1.08. The antibody titer was subsequently decreased as the symptoms improved. Similarly to the antibody titer, the Partial Mayo score showed a downward trend.

### <Example 7: Evaluation of severity of irAE colitis>

The clinical characteristics of the 26 irAE enteritis patients (8 antibody-positive patients and 18 antibody-negative patients) who underwent the antibody test in Example 5 were compared. The irAE enteritis patients were cancer patients who had treatment with an immune checkpoint inhibitor (ICI). After the onset of enteritis, the patients had discontinuation of the ICI and received conservative treatment (ICI discontinuation only), administration of a steroid, or administration of a steroid and an anti-TNFα antibody (infliximab). The severity of adverse events including enteritis was evaluated based on the Common Terminology Criteria for Adverse Events (CTCAE) version 5.0 published by the Cancer Therapy Evaluation Program (CTEP) of the National Cancer Institute (NCI) in the United States in November 2017. A Japanese translation of CTCAE provided by the Japan Clinical Oncology Group (JCOG), "CTCAE v5.0 - JCOG," is shown in Table 2. Results are summarized in Table 3.

**[Table 2]**

| CTCAE | | | | | |
|---|---|---|---|---|---|
| Colitis | Grade 1 | Grade 2 | Grade 3 | Grade 4 | Grade 5 |
| | No symptoms; clinical findings or examination findings only | Abdominal pain; mucous stool, bloody stool | Advanced, persistent abdominal pain; fever, ileus, peritoneal irritation sign | Life-threatening; urgent treatment is needed | Death |

**[Table 3]**

| | | **Anti-integrin αvβ6 antibodies** | | |
|---|---|---|---|---|
| | | **Positive** | **Negative** | **P-value** |
| **Number of patients, n** | | 8 | 18 | |
| Median age, years (range) | | 69 (57-82) | 70.5 (47-80) | 0.404 |
| Sex | | | | |
| | Male, n (%) | 5 (62.5) | 13 (72.2) | 0.667 |
| | Female, n (%) | 3 (37.5) | 5 (27.8) | |

| **Cancer type, n (%)** | | | | |
|---|---|---|---|---|
| Non-small cell lung cancer | | 2 (25) | 6 (33.3) | |
| Melanoma | | 3 (37.5) | 4 (22.2) | |
| Kidney cancer | | 1 (12.5) | 4 (22.2) | |
| Esophageal cancer | | 1 (12.5) | 1 (5.6) | |
| Peritoneal cancer | | 1 (12.5) | 0 (0) | 0.679 |
| Cancer of unknown primary | | 0(0) | 1 (5.6) | |
| Malignant pleural mesothelioma | | 0(0) | 1 (5.6) | |
| Bladder cancer | | 0(0) | 1 (5.6) | |

| **ICI medication, n (%)** | | | | |
|---|---|---|---|---|
| | Pembrolizumab | 4 (50) | 6 (33.3) | |
| | Nivolumab | 3 (37.5) | 5 (27.8) | |
| | Nivolumab and ipilimumab | 0 (0) | 5 (27.8) | 0.465 |
| | Atezolizumab | 1 (12.5) | 1 (5.6) | |
| | Durvalumab | 0 (0) | 1 (5.6) | |

| **Grade ≥3 adverse events, n (%)** | | | | |
|---|---|---|---|---|
| | Diarrhea | 5 (62.5) | 7 (38.9) | 0.401 |
| | Colitis | 6 (75) | 1 (5.6) | <0.001 |

| **Treatment, n (%)** | | | | |
|---|---|---|---|---|
| | Steroid alone | 3 (37.5) | 12 (66.7) | 0.218 |
| | Conservative treatment | 1 (12.5) | 6 (33.3) | 0.375 |
| | Steroid/infliximab | 4 (50) | 0(0) | 0.005 |

| **Prognosis, n (%)** | | | | |
|---|---|---|---|---|
| | Alive | 4 (50) | 12 (66.7) | 0.665 |
| | Dead | 4 (50) | 6 (33.3) | |

As is clear from Table 3, six out of the eight antibody-positive patients (75%) and one out of the 18 antibody-negative patients (5.6%) had severe enteritis (enteritis having CTCAE grade 3 or higher). Thus, severe enteritis was frequently observed in antibody-positive patients.

Regarding treatment, four out of the eight antibody-positive patients were not improved by steroid treatment alone and received additional administration of infliximab. All of the four patients developed severe colitis of grade 3 or higher, and the colitis symptoms were improved by the additional administration. None of the 18 antibody-negative patients had administration of infliximab. In other words, the colitis symptoms of the antibody-negative patients were improved by administration of steroid alone or conservative treatment, and thus additional administration of infliximab was not required.

Based on the above results, it was found that when patients with irAE colitis are positive for anti-integrin αvβ6 antibodies, the colitis tends to be severe and be difficult to treat. Thus, it was shown that measuring the antibody may be useful in the therapeutic management of irAE enteritis.

## Claims

1. A method of testing for irAE enteritis, the method comprising:
detecting an antibody that immunologically reacts with a fragment or an entirety of integrin αvβ6 in a sample as an indicator of ulcerative colitis-like irAE enteritis.

2. The method according to claim 1, wherein the fragment or entirety of integrin αvβ6 is used as an antigen and an antibody that immunologically reacts with the antigen is detected.

3. The method according to claim 1 or 2, wherein the sample is a blood sample.

4. The method according to any one of claims 1 to 3, wherein the indicator is an indicator of severe ulcerative colitis-like irAE enteritis.

5. A kit for testing for irAE enteritis, comprising a fragment or an entirety of integrin αvβ6.

6. The kit according to claim 5, further comprising an antibody for detection.

7. The kit according to claim 5 or 6, further comprising a positive standard solution and/or a negative standard solution.

8. The kit according to any one of claims 5 to 7, wherein the fragment or entirety of integrin αvβ6 is immobilized on a solid phase.

9. The method according to any one of claims 1 to 4, wherein the method is for determining whether or not enteritis developed in a patient receiving immune checkpoint inhibitor (ICI) treatment is ulcerative colitis-like irAE enteritis,
wherein the sample is derived from a patient receiving ICI treatment, and when an antibody titer of the antibody that immunologically reacts with a fragment or an entirety of integrin αvβ6 detected in the sample is higher than an antibody titer of the antibody detected in a control sample, the enteritis developed in the patient is determined to be ulcerative colitis-like irAE enteritis.

10. The method according to claim 9, wherein the patient is a patient receiving combined treatment with an ICI and a cancer chemotherapeutic drug other than an ICI.

11. The method according to any one of claims 1 to 4, wherein the method is for predicting a risk of developing ulcerative colitis-like irAE enteritis in a patient,
wherein the sample is derived from a patient, and when an antibody titer of the antibody that immunologically reacts with a fragment or an entirety of integrin αvβ6 detected in the sample is higher than an antibody titer of the antibody detected in a control sample, the patient is determined to be at risk of developing ulcerative colitis-like irAE enteritis.

12. The method according to claim 11, wherein the patient is a patient prior to ICI treatment or a patient receiving ICI treatment.

13. A method of testing for irAE enteritis, the method comprising:
detecting an antibody that immunologically reacts with a fragment or an entirety of integrin αvβ6 in a sample as an indicator of enteritis for which discontinuation of an ICI is recommended.

14. The method according to claim 13, wherein the indicator is an indicator of enteritis for which discontinuation of an ICI and administration of one or more immunosuppressants are recommended.

15. The method according to claim 13 or 14, wherein the method is for determining whether or not enteritis developed in a patient receiving ICI treatment is enteritis for which discontinuation of an ICI is recommended,
wherein the sample is derived from a patient receiving ICI treatment, and when an antibody titer of the antibody that immunologically reacts with a fragment or an entirety of integrin αvβ6 detected in the sample is higher than an antibody titer of the antibody detected in a control sample, the enteritis developed in the patient is determined to be enteritis for which discontinuation of an ICI is recommended.

16. The method according to claim 13 or 14, wherein the method is for predicting a risk of developing enteritis for which discontinuation of an ICI is recommended in a patient,
wherein the sample is derived from a patient, and when an antibody titer of the antibody that immunologically reacts with a fragment or an entirety of integrin αvβ6 detected in the sample is higher than an antibody titer of the antibody detected in a control sample, the patient is determined to be at risk of developing enteritis for which discontinuation of an ICI is recommended.
